# EUROPEAN PATENT APPLICATION

(11) **EP 2 554 654 A1**
(43) Date of publication of application: **06.02.2013**
(21) Application number: 11762217.5
(22) Date of filing: 28.03.2011
(51) Int. Cl.: C12M 1/34, G01N 27/28, G01N 27/30, G01N 27/416

(54) **SENSOR DEVICE**

(30) Priority: 30.03.2010 JP 2010078444
(71) Applicant: Panasonic Corporation, Kadoma-shi Osaka 571-8501 (JP)
(72) Inventor: YAMAMOTO, Takeki, Chuo-ku, Osaka-shi, Osaka 540-6207 (JP); NAKATANI, Masaya, Chuo-ku, Osaka-shi, Osaka 540-6207 (JP); USHIO, Hiroshi, Chuo-ku, Osaka-shi, Osaka 540-6207 (JP); YAMADA, Yoshiki, Chuo-ku, Osaka-shi, Osaka 540-6207 (JP); NAKANO, Yusuke, Chuo-ku, Osaka-shi, Osaka 540-6207 (JP)
(74) Representative: Vigand, Philippe
(86) International application number: PCT/JP2011/001805
(87) International publication number: WO 2011/121968

(57) **Abstract**

A sensor device includes a mounting substrate, and a sensor chip held on the mounting substrate. The sensor chip includes holding plate (12) and a plurality of through holes (18) each penetrating through a first surface and a second surface of holding plate (12). A solution including a specimen is allowed to flow over the first surface of holding plate (12) and the specimen is held in plurality of through holes (18). The directions of line segments connecting the centers of the adjacent through holes among plurality of through holes (18) are not parallel to a flow direction of the solution.

## Description

### TECHNICAL FIELD

The present invention relates to a sensor device such as a cell electrophysiological sensor adsorbing and holding a specimen such as cells or biological materials and measuring an electrophysiological activity of the specimen, or a substance identification sensor adsorbing and holding a specimen on, for example, a bead that includes a probe and identifying a sample such as a chemical substance, a biological substance or an environment substance.

### BACKGROUND ART

Recently, much attention has been paid to a biosensor and a biochip. The biosensor and the biochip are used for biosensing protein, genes, low-molecular weight signal molecules, or the like, based on the molecular recognition mechanism of a living body. In order to do so, biosensing is carried out by monitoring selection-specific binding such as a receptor - ligand or antigen - antibody reaction, a selection catalytic reaction of, for example, an enzyme by using a predetermined sensor device.

Sensor chips using fine processing technology are being developed for the purpose of applying them to sensor devices that carry out such biosensing. Among them, much attention has been paid to a technique for allowing a solution including a specimen such as a cell or a bead to flow over the upper surface of a plate having a minute through hole, and carrying out electrophysiological measurement or measurement using a fluorescent molecule in a state in which the specimen is held in the through hole and fixed therein.

For example, as a method of electrophysiologically measuring an ion channel that is present in a cell membrane, a cell electrophysiological sensor has been proposed. Unlike a conventional patch clamp technique, this does not require skilled work of introducing a micropipette into each cell, and is suitable for an automated system with high throughput.

In this cell electrophysiological sensor, biological variability (i.e., variation of states, sizes, expression levels of channel in cells) is a main cause of reduction in the success rate in a planer (plane) patch clamp system.

In order to prevent the success rate from being lowered, a sensor device is designed such that one type of specimen is certainly dispensed into a plurality of wells (for example, four wells) to be measured, so that measurement of at least one well succeeds.

For example, a technique for measuring an averaged ionic current from a cell group has been proposed. This technique uses a sensor device in which a plurality of through holes are aligned in lattice in each well of a plate. By carrying out measurement by using such a sensor device, the averaged ionic current measured from the cell group is very uniform and has less variation among wells, and the success rate of measurement is extremely high. As a result, it is not necessary to carry out measurement in a plurality of wells, and the throughput can be improved.

Thus, development of a sensor chip capable of obtaining measurement data that are uniform and have less variation and therefore obtaining a high success rate by forming a plurality of through holes in each well has been desired.

However, when a plurality of through holes are formed in each well, specimens flowing over the upper surface of a plate interfere with each other during measurement, and suction or adsorption of the specimens cannot be easily carried out. Thus, the success rate of adsorption of specimens is lowered, and a gigaseal state cannot be satisfied. As a result, improvement of the success rate of measurement is inhibited.

Note here that examples of prior art mentioned above include the following patent literatures and non-patent literature.

### [Citation List]

### [Patent Literatures]

PTL 1: WO 02/055653
PTL 2: WO 2007/132769

### [Non-Patent Literature]

NPL1: Alan Finkel, and five others, "Population Patch Clamp Improves Data Consistency and Success Rates in the Measurement of ionic Currents," Journal of Biomolecular Screening, 2006, P488-496

### SUMMARY OF THE INVENTION

The present invention facilitates adsorption and holding of a specimen, and improves a success rate of measurement.

The present invention provides a sensor device including a mounting substrate, and a sensor chip held on the mounting substrate. The sensor chip includes a holding plate, and a plurality of through holes each penetrating through a first surface and a second surface of the holding plate. A solution that includes a specimen is allowed to flow over the first surface of the holding plate, and the specimen is held in the plurality of through holes. Directions of line segments connecting the centers of adjacent through holes among the plurality of through holes are not parallel to a flow direction of the solution.

Thus, in the sensor chip provided with a plurality of through holes, specimens can be prevented from interfering with each other, and thereby suction and adsorption of the specimen can be carried out easily. As a result, the success rate of measurement can be improved.

### BRIEF DESCRIPTION OF THE DRAWINGS

Fig. 1 is a schematic sectional view showing a configuration of a cell electrophysiological sensor as an example of a sensor device in accordance with a first exemplary embodiment of the present invention.
Fig. 2 is a sectional view of a sensor chip of the sensor device in accordance with the first exemplary embodiment of the present invention.
Fig. 3 is a schematic plan view of a holding plate of the sensor device in accordance with the first exemplary embodiment of the present invention.
Fig. 4 is a view for illustrating a flow direction of a solution in the sensor device in accordance with the first exemplary embodiment of the present invention.
Fig. 5 is a schematic plan view showing another configuration of the holding plate of the sensor device in accordance with the first exemplary embodiment of the present invention.
Fig. 6 is a view for illustrating a method of manufacturing the sensor chip of the sensor device in accordance with the first exemplary embodiment of the present invention.
Fig. 7 is a view for illustrating the method of manufacturing the sensor chip of the sensor device in accordance with the first exemplary embodiment of the present invention.
Fig. 8 is a view for illustrating the method of manufacturing the sensor chip of the sensor device in accordance with the first exemplary embodiment of the present invention.
Fig. 9 is a view for illustrating the method of manufacturing the sensor chip of the sensor device in accordance with the first exemplary embodiment of the present invention.
Fig. 10 is a sectional view showing a configuration in a vicinity of through holes of the sensor chip of the sensor device in accordance with the first exemplary embodiment of the present invention.
Fig. 11A is a view showing a cleavage plane of a silicon (100) substrate used for the holding plate of the sensor device in accordance with the first exemplary embodiment of the present invention.
Fig. 11B is a view showing a cleavage plane of a silicon (110) substrate used for the holding plate of the sensor device in accordance with the first exemplary embodiment of the present invention.
Fig. 11C is a view showing a cleavage plane of a silicon (111) substrate used for the holding plate of the sensor device in accordance with the first exemplary embodiment of the present invention.
Fig. 12 is a sectional view showing a configuration of a sensor chip of a sensor device in accordance with a second exemplary embodiment of the present invention.
Fig. 13 is a sectional plan view of a holding plate of the sensor chip of the sensor device in accordance with the second exemplary embodiment of the present invention.
Fig. 14A is a plan view showing a configuration of a through hole in the sensor device in accordance with the second exemplary embodiment of the present invention.
Fig. 14B is a sectional view showing a configuration of the sensor device in accordance with the second exemplary embodiment of the present invention.
Fig. 14C is a bottom view showing a configuration of a recess portion of the sensor device in accordance with the second exemplary embodiment of the present invention.
Fig. 15A is a schematic plan view for illustrating a configuration of the sensor chip of the sensor device in accordance with the second exemplary embodiment of the present invention.
Fig. 15B is a schematic plan view for illustrating a configuration of another example of the sensor device in accordance with the second exemplary embodiment of the present invention.
Fig. 16 is a view for illustrating a method of manufacturing the sensor chip of the sensor device in accordance with the second exemplary embodiment of the present invention.
Fig. 17 is a view for illustrating the method of manufacturing the sensor chip of the sensor device in accordance with the second exemplary embodiment of the present invention.
Fig. 18 is a view for illustrating the method of manufacturing the sensor chip of the sensor device in accordance with the second exemplary embodiment of the present invention.
Fig. 19 is a view for illustrating the method of manufacturing the sensor chip of the sensor device in accordance with the second exemplary embodiment of the present invention.
Fig. 20 is a schematic sectional view showing a configuration of a sensor device in accordance with a third exemplary embodiment of the present invention.

### DETAILED DESCRIPTION OF THE PREFERRED EMBODIMENT

Hereinafter, sensor devices in accordance with exemplary embodiments of the present invention are described. Note here that the same reference numerals are given to the same configuration of preceding exemplary embodiments, and detailed description thereof may be omitted. Furthermore, the present invention is not necessarily limited to the below mentioned exemplary embodiments.

### FIRST EXEMPLARY EMBODIMENT

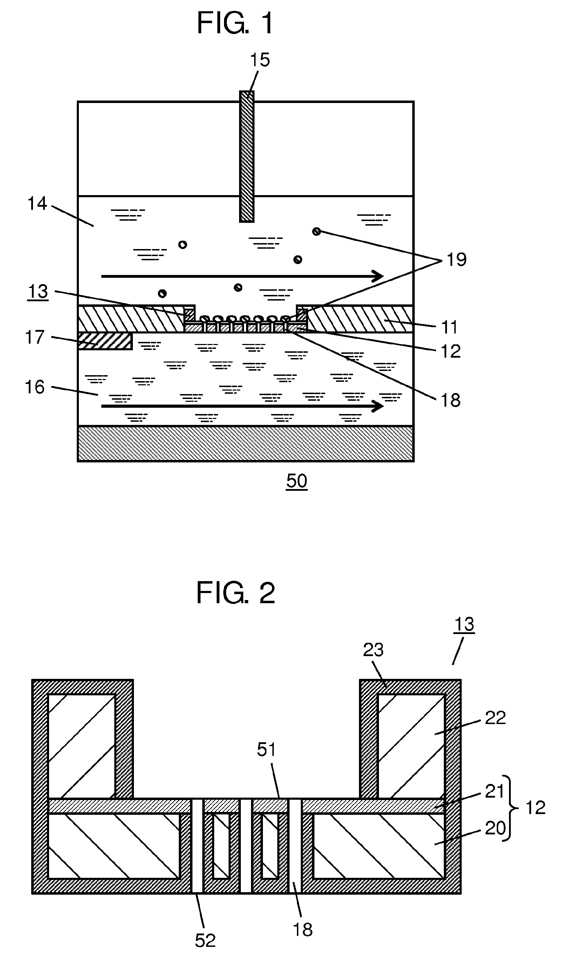

Fig. 1 is a schematic sectional view showing a configuration of cell electrophysiological sensor 50 as an example of a sensor device in accordance with a first exemplary embodiment of the present invention.

As shown in Fig. 1, cell electrophysiological sensor 50 in this exemplary embodiment includes mounting substrate 11, and sensor chip 13 which is mounted on mounting substrate 11 and has holding plate 12. Cell electrophysiological sensor 50 further includes first electrode bath 14 disposed above sensor chip 13 and first electrode 15 disposed inside of first electrode bath 14 and at an upper surface (first surface 51) side of sensor chip 13.

Furthermore, cell electrophysiological sensor 50 includes second electrode bath 16 disposed below sensor chip 13, and second electrode 17 disposed on the lower surface (second surface 52) of sensor chip 13 opposite side to the first surface side inside second electrode bath 16. Sensor chip 13 includes a plurality of through holes 18 formed from the first surface toward the second surface.

First electrode bath 14 is filled with a first electrolytic solution (extracellular fluid) including a cell as specimen 19, and second electrode bath 16 is filled with a second electrolytic solution (intracellular fluid). The first electrolytic solution and the second electrolytic solution flow in the direction shown by arrows shown in Fig. 1.

In this configuration, sensor chip 13 is a boundary interface between the first electrolytic solution (extracellular fluid) and the second electrolytic solution (intracellular fluid). Furthermore, by pressurizing from the first surface side of sensor chip 13 or depressurizing from the second surface side of sensor chip 13 via through holes 18, specimen 19 and the first electrolytic solution are attracted to through hole 18. Thus, specimen 19 is sucked and held so as to block through hole 18, so that it can be assayed.

In this exemplary embodiment, a mammalian muscle cell is used as specimen 19, an electrolytic solution including about 155 mM K⁺ ion, about 12 mM Na⁺ ion and about 4.2 mM Cl⁻ ion is used as the first electrolytic solution, and an electrolytic solution including about 4 mM K⁺ ion, about 145 mM Na⁺ ion, and about 123 mM Cl⁻ ion is used as the second electrolytic solution.

Note here that the first electrolytic solution and the second electrolytic solution which have the same composition may be used.

Next, further suction is carried out from second electrode bath 16 side, or a medicine (for example, Nystatin) is injected, thereby forming micropores in specimen 19.

Thereafter, action that would be stimulation to specimen 19 is carried out from first electrode bath 14 side. Examples of the stimulation include physical stimulation by, for example, mechanical displacement, light, heat, electricity, and electromagnetic wave, in addition to chemical stimulation with, for example, chemical agents or toxic agents.

When specimen 19 actively reacts with respect to such stimulation, specimen 19 releases or absorbs various ions through, for example, a channel of the cell membrane. Thus, since intracellular and extracellular potential gradients are changed, the electric change is detected by first electrode 15 and second electrode 17, and thus a pharmacological reaction of cells can be examined.

In this exemplary embodiment, a mammalian muscle cell is used as an example of specimen 19, but specimen 19 is not necessarily limited to a cell. Specimen 19 may be arbitrary substances such as particles. The above-mentioned configuration of cell electrophysiological sensor 50 can be used widely in fields of agriculture, medicine, and environment and the like, and used in, for example, DNA sensors for detecting DNA sequences for identifying viruses, food production places, or the like, SNP sensors for detecting SNP (single nucleotide polymorphism) sequences, antigen sensors for detecting the presence of allergen (an allergy antigen), or the like.

Next, a structure of sensor chip 13 is described.

Fig. 2 is a sectional view of sensor chip 13 of the sensor device in accordance with the first exemplary embodiment of the present invention.

As mentioned above, sensor chip 13 includes holding plate 12 having silicon dioxide layer 21 on the first surface side, and holding plate 12 is provided with a plurality of through holes 18 communicating between the first surface and the second surface.

It is preferable that holding plate 12 has a laminated structure including silicon layer 20 including silicon as a main component and silicon dioxide layer 21. Furthermore, it is desirable that silicon layer 22 including silicon as a main component is formed on the first surface side of silicon dioxide layer 21 of holding plate 12 so as to surround a plurality of through holes 18. It is desirable because the strength of sensor chip 13 itself can be enhanced.

Furthermore, when silicon layers 20 and 22 are covered with insulating film 23 such as a silicon dioxide film or a silicon nitride film, measurement accuracy can be improved. In particular, it is preferable that electrical measurement noise can be reduced in use as cell electrophysiological sensor 50.

As a base material for producing sensor chip 13, it is desirable to use a SOI (Silicon on Insulator) substrate whose silicon layer 20 includes a silicon (100) plane. The SOI substrate has a three-layered structure of silicon layer - silicon dioxide layer - silicon layer.

When the SOI substrate is used and fine processing is carried out by using photolithography and etching technique, a large number of precise sensor chips 13 can be produced at one time. When the SOI substrate is used, since silicon dioxide layer 21 functions as an etching stop layer in an etching process, sensor chip 13 can be produced with high accuracy.

Furthermore, since silicon dioxide layer 21 is extremely hydrophilic, generation of air bubbles at the time of measurement can be suppressed and air bubbles can be removed easily. This permits highly accurate measurement. At the time of measurement, if air bubbles remain in the vicinity of through holes 18, a gigaseal property is largely reduced, and the measurement accuracy may be adversely affected. It is preferable that the thickness of silicon dioxide layer 21 is 0.5 to 10 µm from the viewpoint of the thickness required for the etching stop layer and productivity.

The thickness of holding plate 12 having a plurality of through holes 18 may be several µm, and, therefore, silicon layer 22 is formed with the handling and the mounting property in manufacturing process taken into consideration. Therefore, silicon layer 22 functions as a holding portion of sensor chip 13, and plays a role of keeping the mechanical strength and has a function of storing a liquid.

Note here that silicon layer 22 is not an indispensable component, and a predetermined dimension thereof can be appropriately selected according to the shape and structure of sensor chip 13. For formation of silicon layer 22, a method of etching using the SOI substrate, bonding, and the like, are considered. From the viewpoint of the consistency of process, etching from the SOI substrate is preferable.

When the thickness of holding plate 12 is increased, cracking does not easily occur, but time necessary for processing becomes longer. Therefore, from the viewpoint of the throughput of steps, the thickness of holding plate 12 is desired to be thin. When the thickness of holding plate 12 is increased, the length of through hole 18 is increased. As a result, resistance of a flow passage when specimen 19 is adsorbed by a pressure becomes large, and specimen 19 cannot be sucked easily. Consequently, the success rate of the measurement is reduced. From such viewpoints, the thickness of holding plate 12 is desirably about 5 to 50 µm.

In the above-mentioned example, for the base material for producing sensor chip 13, the SOI substrate whose silicon layer 20 includes silicon (100) is selected. However, it is possible to use a silicon (110) substrate, a silicon (111) substrate, or silicon substrates having other plane orientations, a glass substrate, film resin, or the like. In such a case, the shape of sensor chip 13 and arrangement of through holes 18 may be employed such that the same effect as in this exemplary embodiment can be obtained.

From the viewpoint of workability and versatility, it is preferable that a substrate including silicon (100) is used as the base material. The substrate including silicon (100) may include at least silicon (100) in the substrate. That is to say, not only a substrate made of a simple substance of silicon (100), but also a SOI substrate in which silicon (100) is used as silicon layer 20, a substrate partially or entirely doped with an element such as boron, or a substrate formed by bonding silicon (100) onto glass or the like, may be used.

Note here that silicon dioxide layer 21 that functions as an etching stop layer is generally formed by thermal oxidation, but it can be formed by using silicon dioxide layer 21 formed by a CVD (Chemical Vapor Deposition) method, a sputtering method, a CSD (Chemical Solution Deposition) method, or the like. Furthermore, doped-oxide layers such as a so-called PSG (Phosphorus Silicon Glass) layer doped with phosphorus, a so-called BSG (Boron Silicon Glass) layer doped with boron, or a BPSG (Boron Phosphorus Silicon Glass) layer doped with phosphorous and boron may be used.

Next, the shape of sensor chip 13 and arrangement of through holes 18 are described in detail.

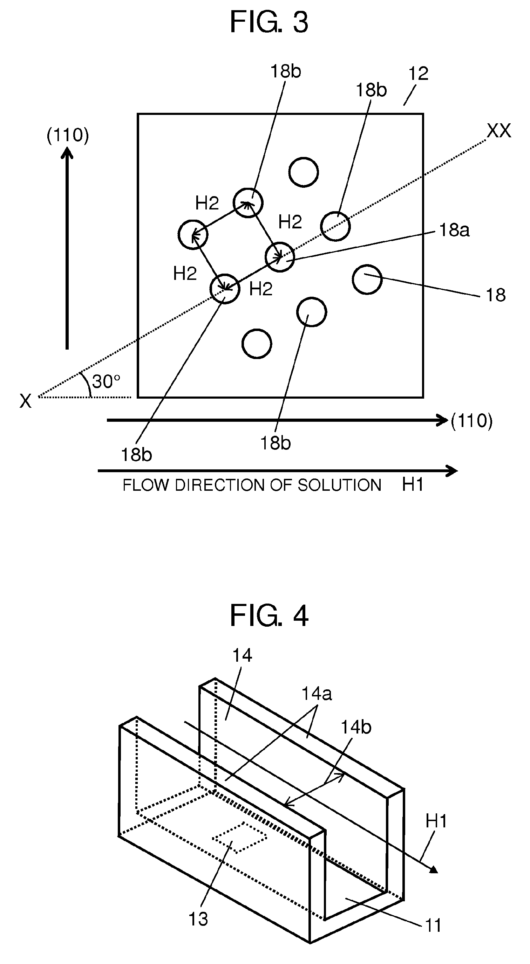

Fig. 3 is a schematic plan view of holding plate 12 of the sensor device in accordance with the first exemplary embodiment of the present invention.

Fig. 3 shows arrangement of through holes 18 formed on holding plate 12 of sensor chip 13. An arrow in the lower part of Fig. 3 shows flow direction H1 of a solution flowing over the first surface side of sensor chip 13. In sensor chip 13 in this exemplary embodiment, a plurality of through holes 18 are formed such that solution flow direction H1 is not parallel to the directions of line segments H2 connecting the centers of adjacent through holes 18 (four directions in Fig. 3).

Herein, adjacent through holes 18 denote the most adjacent two through holes. For example, through holes adjacent to through hole 18a are through holes 18b located most adjacent in distance between the center of the through holes and the center of through hole 18a.

Furthermore, solution flow direction H1 denotes a flow direction of fluid as an average.

Fig. 4 is a view for illustrating solution flow direction H1 in the sensor device in accordance with the first exemplary embodiment of the present invention.

As shown in Fig. 4, when wall surfaces 14a of first electrode bath 14 are parallel to each other and flow passage width 14b of the first electrode bath is constant, solution flow direction H1 is a direction parallel to wall surface 14a.

On the other hand, when wall surfaces 14a of first electrode bath 14 are not parallel to each other, and when flow passage width 14b of first electrode bath 14 is not constant, that is to say, when the flow passage is gradually narrower along the direction in which a solution flows or when it is gradually wider along the direction in which a solution flows, solution flow direction H1 is the flow direction of fluid as an average.

In this exemplary embodiment, as shown in Fig. 3, the shape of holding plate 12 of sensor chip 13 is a square, and nine through holes 18 are formed. Furthermore, nine through holes 18 are arranged such that line segment H2 drawn by connecting centers of adjacent through holes 18 forms a square. Furthermore, an extended line of line segment H2 connecting the centers of adjacent through holes 18 (a line shown by X-XX in Fig. 3) rotates at 30° with respect to one side (the lower side in Fig. 3) of the square shape of holding plate 12.

As mentioned above, it is desirable that the shape of holding plate 12 of sensor chip 13 is a square. This is because a waste region on which through holes 18 are not formed in holding plate 12 of sensor chip 13 can be minimized, and holding plate 12 can be used efficiently, contributing to the reduction of cost.

It is preferable that a silicon (100) plane is used as a silicon layer of sensor chip 13. Furthermore, it is more preferable that sensor chip 13 has a square shape including a silicon (110) direction in one side. When sensor chip 13 is shaped in a square shape including the silicon (110) direction, which is a cleavage plane, of silicon in one side, when sensor chip 13 is separated by conventional blade dicing, excellent workability is obtained. Note here that in this exemplary embodiment, holding plate 12 is formed in a 1 mm x 1 mm square shape.

The shape of holding plate 12 of sensor chip 13 is not necessarily limited to a square, and the square shape does not necessarily include the silicon (110) direction in one side. Other examples of the shape of holding plate 12 of sensor chip 13 include a circular shape, a parallelogram, and a rectangular shape.

When the processed surface is a surface other than the silicon (110) plane, workability by blade dicing is reduced. However, when dicing with a laser and etching between sensor chips 13 are used, workability of sensor chip 13 is improved and arbitrary shapes can be selected. Use of dicing with a laser makes it possible to carry out processing with a high speed, less chipping, and high transverse rupture strength, and therefore, the thickness and size of sensor chip 13 can be reduced. Processing by laser dicing can be discriminated by thermal effect, debris contamination, occurrence of cracks perpendicular to the direction of sensor chip 13 surface, or the like.

Furthermore, a scallop may be formed on the side surface of sensor chip 13. When a plurality of sensor chips 13 are simultaneously formed by using one substrate, etching can be used when sensor chip 13 is separated individually. In this case, a trace of etching is generated on the side surface of sensor chip 13. In particular, when a Bosch process of repeating a cycle including etching and deposition is used, a stepped shape called scallop is generated on the side surface of sensor chip 13. The step of the scallop is in the order of several nm to several tens nm.

The number of through holes 18 may be selected arbitrarily. This can be formed by the similar processes by changing the number of mask holes in a resist mask formed when through holes 18 are formed.

When the number of through holes 18 is increased, since an averaged ionic current can be measured, the success rate of the measurement is improved. However, when the number of through holes 18 is too large, through holes 18 in which specimen 19 is not held are easily are generated, so that the synthesized resistance of through sensor chip 13 as a whole is reduced and appropriate measurement of ionic current cannot be carried out.

In general, the number of through holes 18 can be 2 to 128. However, when the success rate of measurement is taken into consideration, it is desirable that the number of through holes 18 is about 10. In this exemplary embodiment, in order to increase the arrangement density of through holes 18, nines through holes 18 are formed in such a manner that a shape formed by connecting the centers of nine (3 x 3) through holes 18 is a square. The arrangement of through holes 18 can be selected appropriately from the shape of sensor chip 13 and the number of through holes 18.

Note here that it is desirable that the distance between the centers of through holes 18 is not less than 20 µm from the viewpoint of success rate of measurement. Since the size of a cell used as specimen 19 is about 20 µm although depending upon the types of specimens 19, culture conditions, or the like, when the interval between through holes 18 is smaller than this size, interference between cells occurs and the success rate of measurement is reduced.

Since structurally weak portions do not easily aggregate by increasing the distance between through holes 18, the reliability of sensor chip 13 is improved. However, if a large number of holes are necessary, a region necessary for forming through holes 18 is increased, and thus it is difficult to reduce the size of the sensor device and to reduce a cost.

In this exemplary embodiment, in order to prevent the interference between specimens 19 by providing a sufficient distance, the distance between through holes 18 is set at 50 µm.

It is preferable that through holes 18 are arranged in an equal interval. It is desirable that surrounding through holes 18 are arranged bilaterally and vertically symmetrical with respect to one through hole 18a. That is to say, through holes 18 are arranged point symmetrical with respect to the central point of holding plate 12. By arranging through holes 18 in this way, symmetry occurs when specimens 19 are adsorbed, and averaged data can be obtained.

In this exemplary embodiment, as mentioned above, through holes 18 are arranged such that line segment H2 (the line shown by X-XX in Fig. 3) connecting the centers of adjacent through holes 18a and 18b rotates at 30° with respect to the silicon (110) direction used as holding plate 12, but the similar effect can be obtained at any angles. However, when the angle is, for example, 45°, since the centers of through holes 18 are aligned on the silicon (110) direction used as holding plate 12, in through holes 18 arranged in the direction, interference of specimens 19 flowing into the flow passage may occur. Therefore, it is desirable to select any other angles in which the centers of through holes 18 are not aligned in the silicon (110) direction.

A shape drawn by line segments H2 connecting the centers of adjacent through holes 18 may be shapes other than a square. Through holes 18 may be arranged such that the shape drawn by line segments H2 connecting the centers of adjacent though holes 18 is a regular polygon, or may be arranged at random.

However, in order to improve the symmetry when specimens 19 are adsorbed or to improve the density of through holes 18 in sensor chip 13, it is preferable that the shape drawn by line segments H2 is a regular polygon.

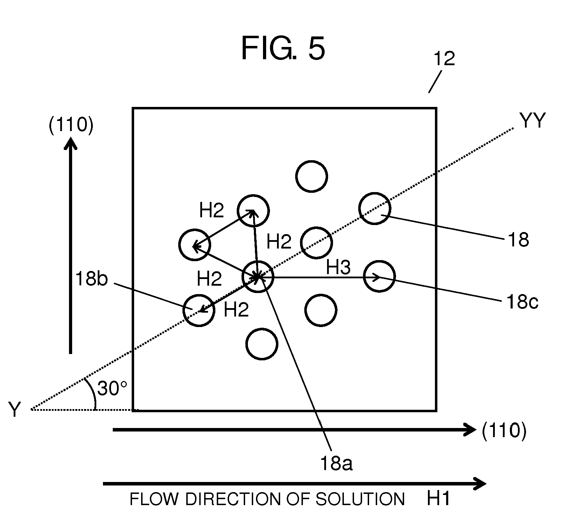

Fig. 5 is a schematic plan view showing another configuration of holding plate 12 of the sensor device in accordance with the first exemplary embodiment of the present invention.

In holding plate 12 of sensor chip 13 shown in Fig. 5, through holes 18 are formed such that a shape drawn by line segments H2 by connecting the centers of adjacent through holes 18 is a regular triangle. At this time, solution flow direction H1 is not parallel to line segment H2 connecting the centers of adjacent through holes 18a and 18b. Note here that solution flow direction H1 is parallel to line segment H3 connecting the centers of not-adjacent through holes 18a and 18c, and the relation between the length of line segment H3 connecting the centers of adjacent through holes 18a and 18c and line segment H2 connecting the centers of adjacent through holes 18a and 18b need to satisfy the relation: H3 > H2.

Furthermore, through holes 18 are arranged such that line segment H2 (a line shown by Y-YY in Fig. 5) connecting the centers of adjacent through holes 18a and 18b is rotated at 30° with respect to the silicon (110) direction used as holding plate 12.

Also when through holes 18 as shown in Fig. 5 are formed, the size of sensor chip 13 can be reduced, and the density of through holes 18 can be enhanced, thus making it possible to adsorb and hold specimen 19 efficiently.

As mentioned above, according to sensor chip 13 of this exemplary embodiment, the directions of line segments H2 connecting the centers of adjacent through holes 18a and 18b do not correspond to flow direction H1 of a solution flowing over the first surface side. Thus, in the flow direction of a solution including specimen 19, it is possible to prevent a phenomenon that when specimen 19 is held in the front-side through hole 18, specimen 19 held in the front-side through hole 18 hinders holding of specimen 19 by the rear-side though hole 18. In other words, since the possibility at which specimens 19 are held in through holes 18 is increased, the success rate of measurement can be improved.

Next, a method of manufacturing sensor chip 13 of cell electrophysiological sensor 50 as an example of the sensor device in this exemplary embodiment is described.

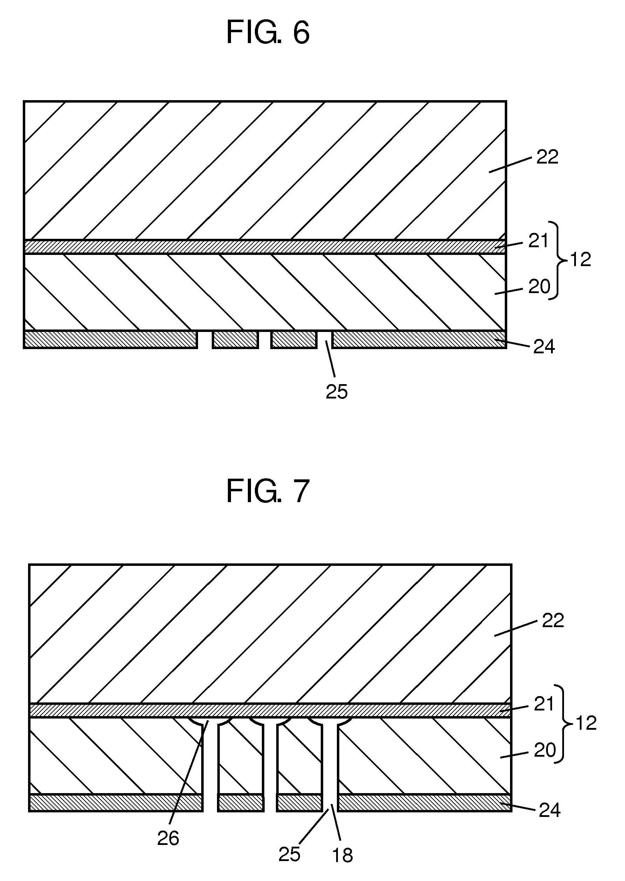

Figs. 6 to 9 are views for illustrating a method of manufacturing sensor chip 13 in the sensor device in accordance with the first exemplary embodiment of the present invention.

Firstly, as a substrate for producing sensor chip 13, the SOI substrate whose silicon layer 20 includes silicon (100) is prepared.

Then, as shown in Fig. 6, first resist mask 24 is formed on the surface (the lower surface in Fig. 6) of silicon layer 20. At this time, with respect to sensor chip 13, a desired number of mask holes 25 having substantially the same shape as the shape of the opening of through hole 18 are patterned.

Next, as shown Fig. 7, through holes 18 are formed by etching silicon layer 20 from mask hole 25 side. An etching process is desirably dry etching because fine processing can be carried out with high accuracy. When dry etching is carried out, in order to form through holes 18 having a high aspect ratio (being deep with respect to the hole diameter), gas for promoting etching (etching gas) and gas for suppressing etching are alternately used.

In this exemplary embodiment, SF₆ is used as the etching gas, and C₄F₈ is used as the suppressing gas. In the dry etching step, plasma is generated by an inductive coupling method of an external coil, and when SF₆ as the etching gas is introduced therein, F radical is generated. Then, the generated F radical is reacted with silicon layer 20 and silicon layer 20 is chemically etched.

At this time, when high frequency is applied to silicon layer 20, a negative bias voltage is generated in silicon layer 20. Then, positive ions (SF₅⁺) included in the etching gas vertically collide with silicon layer 20, and silicon layer 20 is physically etched by the ion bombardment. Thus, dry etching proceeds vertically in silicon layer 20.

On the other hand, suppressing gas C₄F₈ is used, high frequency is not applied to silicon layer 20. Thus, no bias voltage is generated in silicon layer 20. Therefore, CF⁺ included in suppressing gas C₄F₈ is attached to a wall surface of dry etched hole of silicon layer 20 without being biased, a polymerized film made of uniform fluorocarbon is formed on the surface.

This fluorocarbon film as a polymerized film suppresses etching. This polymerized film is formed not only on a wall surface portion of through hole 18 but also on a bottom surface. Since the polymerized film formed on the bottom surface of through hole 18 is removed more easily by ion bombardment as compared with the polymerized film formed on the wall surface, etching proceeds vertically. When such etching proceeds, etching reaches the surface of silicon dioxide layer 21, and processing of the etching in the depth direction stops at an expressed surface of silicon dioxide layer 21.

Note here that as shown in Fig. 7, concave portion 26 can be provided in the vicinity of the interface between silicon layer 20 in through holes 18 and silicon dioxide layer 21. Silicon dioxide layer 21 has a property that it is not easily etched in the above-mentioned etching conditions. Therefore, when etching proceeds and reaches the surface of silicon dioxide layer 21, as mentioned above, the proceeding of etching to the depth direction stops on an expressed surface of silicon dioxide layer 21.

Thereafter, when etching is further carried out, etching ions are accumulated on the expressed surface of silicon dioxide layer 21, and etching ions and the etching ions accumulated on the surface of silicon dioxide layer 21 repel each other, and the etching ions start to proceed in the lateral direction. Accordingly, in the vicinity of silicon dioxide layer 21, concave portion 26 can be formed in a taper shape in which opening width is gradually increased.

In this way, holding plate 12 is formed in a laminated structure of silicon layer 20 as a conductive material and silicon dioxide layer 21 as an insulating material, etching easily proceeds in the lateral direction on the surface of silicon dioxide layer 21, and concave portion 26 can be formed. The depth of concave portion 26 is about 1 µm. The depth can be controlled according to the etching time.

Other examples of the etching gas include CF₄, and other examples of the suppressing gas include CHF₃.

An opening of through hole 18 of holding plate 12 may be provided with a recess portion (not shown). When a recess portion is provided at the first surface side of holding plate 12 that holds specimens 19, since specimens 19 are easily allowed to densely aggregate in the vicinity of through hole 18, the success rate of measurement is improved.

Furthermore, a recess portion is formed at the second surface side of through hole 18 of holding plate 12, the resistance of the flow passage can be suppressed. Thus, even when the thickness of silicon layer 20 is large, the resistance of the flow passage is reduced, and the circulation efficiency of drugs and ease in adsorption or suction of specimen 19 can be achieved.

For formation of the above-mentioned recess portion, from the viewpoint of the mass production, it is preferable to employ wet etching with an alkaline solution. In this case, since the etching speed is generally satisfies: silicon (100) plane > silicon (110) plane >> silicon (111), a pyramid-shaped recess portion is formed.

Furthermore, for formation of the recess portion, etching with gas capable of etching silicon, for example, SF₆, CF₄, Cl₂, and XeF₂, can be used. According to this method, shapes can be controlled variously.

Note here that through hole 18 of holding plate 12 may be provided with a rim portion (not shown). In particular, by providing a rim portion in the position nearer the first surface side of holding plate 12 for holding specimens 19, adhesiveness of specimen 19 can be improved. At this time, it is desirable that the rim portion protrudes substantially perpendicular to the first surface of holding plate 12. However, the same effect can be obtained when the rim portion protrudes substantially parallel to the first surface of holding plate 12, or the rim portion protrudes obliquely with an angle with respect to the first surface of holding plate 12.

Note here that it is preferable that the inner wall of through hole 18 of holding plate 12 is smooth.

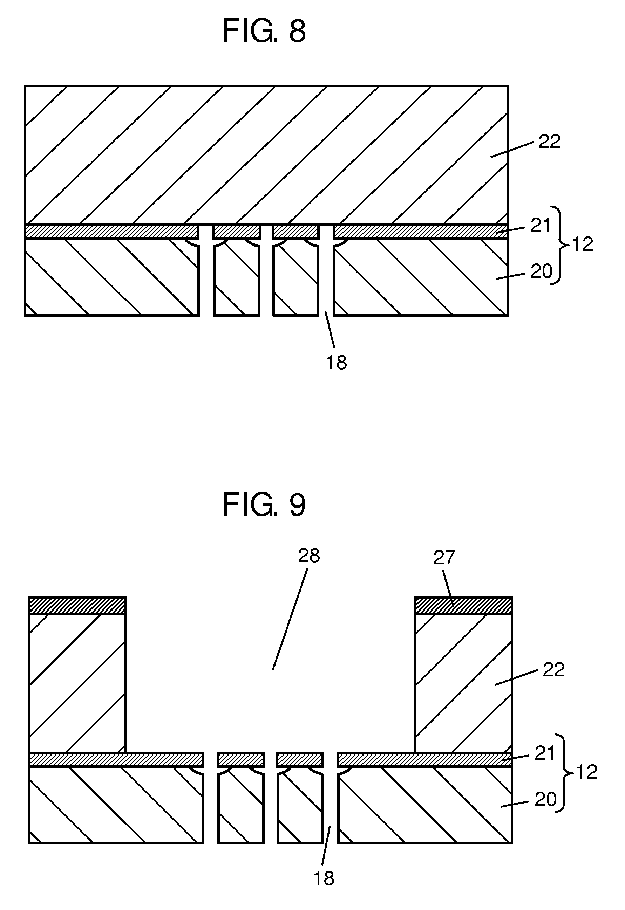

Next, as shown in Fig. 8, silicon dioxide layer 21 is dry-etched from the second surface side. As the etching gas used for dry etching, for example, a mixed gas of CHF₃ and Ar is used. In the mixed gas of CHF₃ and Ar, Ar gas excited with plasma is an etching gas having high straightness. When a large amount of the etching component, for example, an Ar gas, for carrying out sputtering, is used, the opening of through hole 18 straightly advances and enters, and silicon dioxide layer 21 as an insulating material can be selectively etched.

Furthermore, CHF₃ does not easily form a polymerized film on the surface of silicon dioxide layer 21, but forms a polymerized film made of fluorocarbon on the surface of silicon layer 20. Furthermore, a fluorocarbon film when through holes 18 are formed functions as a protective film. Therefore, it is possible to obtain an effect of selectively etching silicon dioxide layer 21 easily. Other examples of the etching gas used at this time include CF₄/H₂, CHF₃/SF₆/He, or the like.

As mentioned above, since two kinds of materials of the laminated body of holding plate 12 have different etching rates with respect to the same gas, respectively, silicon dioxide layer 21 is not etched when silicon layer 20 is etched, and silicon layer 20 is not etched when silicon dioxide layer 21 is etched. By using this property, a desirable shape of through hole 18 can be easily formed.

Next, as shown in Fig. 9, after second resist mask 27 is formed from a surface side (first surface side) of silicon layer 22, silicon layer 22 is etched to form cavity 28 in the same etching conditions as those in which silicon layer 20 is etched. The proceeding of etching in the depth direction also stops at an expressed surface of silicon dioxide layer 21. At this time, as shown in Fig. 9, the periphery of through hole 18 of silicon dioxide layer 21 has a shape in which silicon dioxide layer 21 protrudes (overhang state).

Then, when the product obtained above is placed into a heat treatment furnace in the air, and heated in an atmosphere including oxygen, the surfaces of silicon 20 and 22 are oxidized, and insulating film 23 as a uniform dioxide film is formed on the expressed surfaces of silicon 20 and 22. In this way, sensor chip 13 can be manufactured.

In this exemplary embodiment, the thickness of insulating film 23 is made to be 200 to 230 nm. At this time, strictly speaking, the thickness of silicon dioxide layer 21 is simultaneously increased. However, since oxidization proceeds by diffusion of oxygen, an increased amount of the thickness varies depending upon the thickness of silicon dioxide layer 21 before oxidization. For example, when the thickness of silicon dioxide layer 21 before oxidization is 500 nm, the increased amount of the thickness is about 50 nm.

Furthermore, the formation temperature of insulating film 23 can be made to be not lower than 700°C.

By covering at least a surface of silicon layer 20 expressed on the inner wall surface of through holes 18 with insulating film 23, the capturing property and gigaseal property of a cell as specimen 19 can be improved. However, in the process of thermal oxidation, since sensor chip 13 is placed in a heat treatment furnace, it is preferable from the viewpoint of efficient productivity that the entire expressed surfaces of silicon 20 and 22 are covered with insulating film 23.

In this way, by covering the surfaces of sensor chip 13 with insulating film 23 and silicon dioxide layer 21, the hydrophilicity of sensor chip 13 can be enhanced. Thus, it is possible to achieve sensor chip 13 for cell electrophysiological sensor 50, which is capable of enhancing wettability with a liquid such as a drug solution and suppressing generation of air bubbles.

Furthermore, by covering the surface of sensor chip 13 with an insulating material, electric insulation between first electrode 15 and second electrode 17 can be enhanced, and improvement of measuring accuracy in measuring an electrophysiological phenomenon and reproducibility can be enhanced. This is because an electric signal from the cell membrane is several nA, and therefore when a transient current is generated by stray capacitance, it becomes difficult to carry out highly accurate measurement.

As a method of forming insulating film 23, dry oxidization of carrying out oxidization by introducing only an oxygen gas is used, but the same effect can be obtained by other methods. For example, wet oxidization of sending an oxygen gas and a hydrogen gas at the ratio of 1 : 2 so as to form water vapor (H₂O) in the vicinity of the introduction port of a furnace and sending the formed water vapor to the surface of the silicon surface to be oxidized, or oxidization in the atmosphere in which halogen such as HCl or Cl₂ is added can be employed.

Note here that a material of insulating film 23 for covering silicon layers 20 and 22 is not necessarily limited to a silicon dioxide film similar to silicon dioxide layer 21 functioning as a stop layer. Examples of the material include doped oxide films such as a so-called PSG film doped with phosphorus, a so-called BSG film doped with boron, or a BPSG film doped with phosphorous and boron. Furthermore, a silicon dioxide film formed by other methods such as a CVD method, a sputtering method, and a CSD method may be employed.

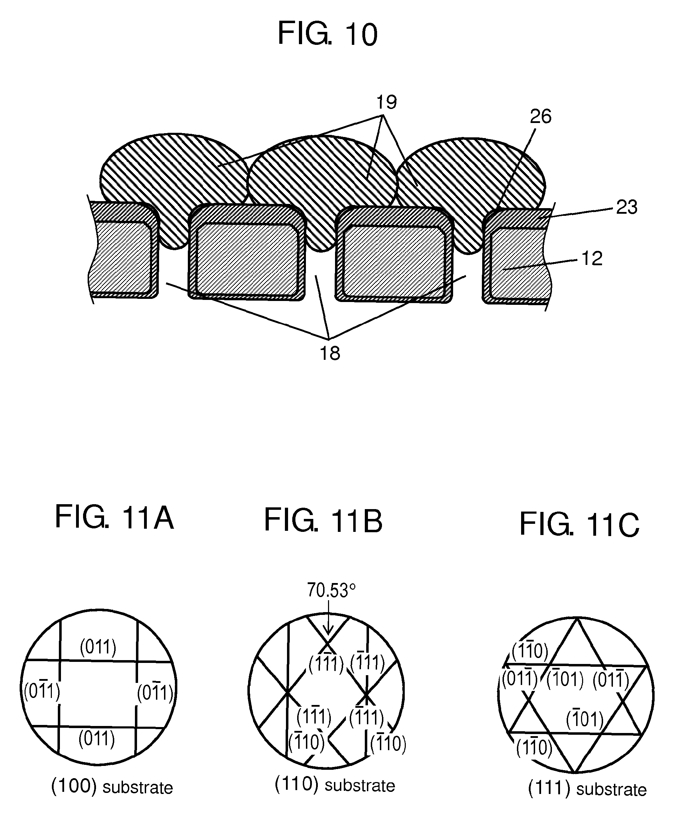

Fig. 10 is a sectional view showing a configuration in the vicinity of through holes 18 of sensor chip 13 of the sensor device in accordance with the first exemplary embodiment of the present invention.

As shown in Fig. 10, insulating film 23 is formed in the periphery of holding plate 12 including the inner wall of through holes 18. This is because when the temperature is kept at not lower than the softening points of silicon dioxide layer 21 and insulating film 23, the surfaces of silicon dioxide layer 21 and insulating film 23 start to be melted and the surface is smoothed (reflow). This is also because the protruding portion of silicon dioxide layer 21 in the overhang state in the periphery of each through hole 18 shown in Fig. 9 flows in such a state in which it hangs on the inner wall surface of through holes 18, and continuously communicates with insulating film 23 formed on the inner wall surface of through holes 18. Thus, sensor chip 13 having a smooth curved surface shape can be produced on through hole 18 toward an opening (a part in which through hole 18 and specimen 19 are brought into contact with each other in Fig. 10).

At this time, holding plate 12 in the periphery of through hole 18 is a surface having an extremely excellent smoothness with the square mean roughness: Rq = not more than 1.0 nm. The square mean roughness Rq is defined by a square root of the square mean roughness of deviation from the mean value to the measurement value when the distribution of the surface roughness is measured.

With the configuration shown in Fig. 10, since specimen 19 is captured along the curved surface of the opening, adhesiveness between specimen 19 and the opening of through hole 18 is enhanced, the adhesion state is easily maintained, and the measurement accuracy by the sensor device can be improved. This is because the opening of through hole 18 is formed by a smooth surface with square mean roughness of Rq = not more than 1.0 nm, and, similarly, a smooth plane portion with square mean roughness of Rq = not more than 1.0 nm is formed on the upper surface of holding plate 12.

A not-doped silicon dioxide formed by thermal oxidation has a relatively high softening temperature, 1160°C. However, the softening temperature of the PSG layer is low, that is, the softening point is about 1000°C; the softening temperatures of the BSG layer and the BPSG layer are low, that is, the softening points are about 900°C. Therefore, when these materials are used, a reflow temperature can be reduced.

When a PSG layer is used, the concentration of phosphorous is about 6 to 8 weight %. When the BPSG layer is used, the dopant concentration is 1 to 4 weight % for boron, and 4 to 6 weight % for phosphorous. When the concentration of phosphorous is higher than about 7 to 8 weight %, phosphorous in the oxide and the water content of the air react with each other. Furthermore, when the concentration of boron is higher than about 4 %, glass is unstable in high humidity.

Furthermore, when a silicon dioxide layer formed by a CVD method is used as insulating film 23, since it has the softening point of around 1000°C, it can be melted by heat of about 1000°C. Consequently, sensor chip 13 achieving energy saving and high accuracy can be formed. The silicon dioxide layer formed by the CVD method has a softening point lower than that of the dioxide layer formed by thermal oxidation. Furthermore, the silicon dioxide layer formed by the CVD method has also self-flattening property by the flowing of a polymerized product on the film surface at temperatures of not lower than 400°C.

As a raw material used in the formation by the CVD method, TEOS-O₃ is particularly excellent in the self-smoothness, but SiH₄-O_{2,} TEOS-O_{2,} or the like, which is a combination of SiH or TEOS and a gas functioning as an oxidizing agent, may be employed.

Whether the silicon dioxide layer is formed by the CVD method or the thermal oxidation can be found by comparing the index of refraction or density. The silicon dioxide formed by the CVD method has a refractive index of about 1.46, and the silicon dioxide formed by thermal oxidation has a refractive index of about 1.48. The refractive indices are values measured by ellipsometry by using a He-Ne laser with a wavelength of 632.8 nm.

Furthermore, since the density of the silicon dioxide layer is difficult to be measured directly, it can be analyzed from the etching rate of buffered hydrofluoric acid (BHF). When BHF (a solution obtained by adding 100 ml of solution, which is obtained by adding 11 g of NH₄F to 680 ml of H₂O, to 10 ml of 48% HF) is used, a silicon dioxide layer by the CVD method has an etching rate of about 20 Å/min, and the silicon dioxide layer by the thermal oxidation is about 6.8 to 7.3 Å/min.

When sensor chip 13 is kept at a temperature that is not lower than the softening point of insulating film 23, the hole shape of through hole 18 approaches a perfect circle, but the shape changes depending upon conditions such as temperatures and time at which sensor chip 13 is kept, and the formation thickness of insulating film 23. As the formation thickness of insulating film 23 is larger, the tendency becomes stronger. Since higher temperatures and longer time cause a large change in the shape, the shape tends to be substantially a perfect circle.

Note here that the shape of through hole 18 is not particularly limited, optimum shapes may be selected depending upon the types, shapes, or the like, of specimens 19 to be measured. However, in the case of cells or spherical particles, since specimens 19 are substantially a perfect sphere, the shape of through hole 18 in this case is desirably nearer to a perfect circle because specimens 19 can be sucked isotropically.

In this exemplary embodiment, because holding plate 12 made of silicon is used, cracking of holding plate 12 can be reduced, and the success rate of measurement can be improved. Unlike film resin which tends to be deformed and in which problems of cracking do not easily occur, a silicon single crystal material is brittle and easily broken and has a cleaving property, so that a specimen holding portion is easily cracked. In particular, cleavage is destruction that occurs in the surface in which a binding force between atoms is weak in a crystal structure, and may easily occur due to a flow, broken, or the like, in the substrate during production process.

However, in this exemplary embodiment, a silicon (100) plane is used for the surface provided with through holes 18 of holding plate 12 such that the centers of adjacent through holes are not aligned in the silicon (110) direction. Thus, since structurally weak through holes 18 are not continuously arranged on a cleavage plane of silicon, cracking of holding plate 12 can be reduced.

When substrates with other orientations, for example, a silicon (110) substrate, a silicon (111) substrate, or the like, are used, the direction in which a cleavage plane is present on the substrate of silicon is different depending upon the orientations of the silicon substrate. Therefore, in order to obtain the same effect, through holes 18 need to be arranged such that the centers of through holes 18 are not aligned on the surface of the cleavage plane.

Figs. 11A to 11C are views showing a cleavage plane of a silicon substrate used in holding plate 12 of the sensor device in accordance with the first exemplary embodiment of the present invention. Fig. 11A shows a cleavage plane of the silicon (100) substrate, Fig. 11B shows a cleavage plane of the silicon (110) substrate, and Fig. 11C shows a cleavage plane of the silicon (111) substrate.

When through holes 18 are formed in such a manner that the direction of each of the cleavage planes shown in Figs. 11A to 11C and the directions of line segments H2 connecting adjacent through holes 18 do not correspond to each other, cracking of holding plate 12 of sensor chip 13 can be reduced. This makes it possible to measure a value of an ionic current of a cell as specimen 19, and to improve the success rate of measurement.

In general, the orientation of the substrate can be discriminated by the X-ray diffraction method (CuKα ray, λ = 0.15418 nm). A diffraction peak occurs at 69.2° when the silicon (100) substrate is used; at 47.3° when the silicon (110) substrate is used; and at 28.5° when the silicon (111) substrate is used. Thus, the orientation of each substrate can be discriminated.

As mentioned above, according to this exemplary embodiment, it is possible to provide a sensor device in which when the silicon (100) substrate is used and plurality of through holes 18 are formed in each sensor chip 13, specimens 19 flowing into the flow passage during measurement are prevented from interfering with each other, and thereby suction and adsorption of cells are easily carried out and a success rate of measurement is high.

In particular, when a base material having the silicon (100) plane for the surface thereof is used, and line segment H2 connecting the centers of adjacent through holes 18a and 18b does not correspond to the silicon (110) direction, cracking of holding plate 12 is reduced, and the success rate of measurement can be improved. This is because the centers of the most adjacent through holes 18 are not aligned on the silicon (110) direction, and structurally weak through holes 18 are not continuously arranged on the cleavage plane of silicon. Consequently, an ionic current value of a cell as specimen 19 can be measured accurately, and therefore improvement of the success rate of measurement can be expected.

Furthermore, even if the distance between the centers of through holes 18 is small, the strength of sensor chip 13 can be maintained, and higher density and smaller size of sensor chip 13 can be achieved. Thus, the number of sensor chips 13 produced from the same area is increased, which can contribute to the reduction of cost of sensor chip 13.

Note here that sensor chip 13 in this exemplary embodiment is not limited to a case in which specimen 19 is introduced by using a flow passage but it can be applied to the case in which specimen 19 is allowed to drop on sensor chip 13.

### SECOND EXEMPLARY EMBODIMENT

Next, a second exemplary embodiment of the present invention is described.

Note here that the same reference numerals are given to the same components described in the first exemplary embodiment and the description thereof is omitted.

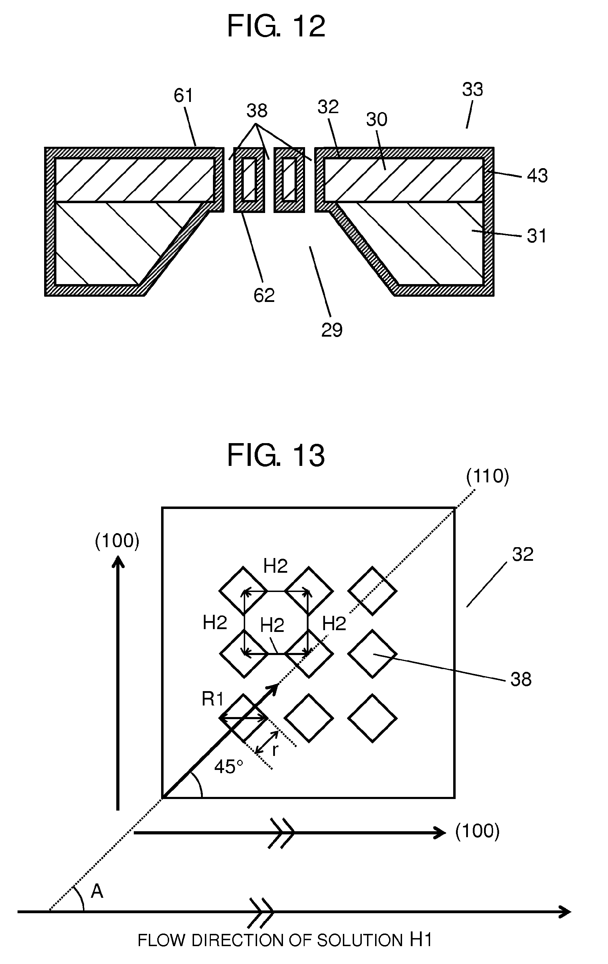

Fig. 12 is a sectional view showing a configuration of sensor chip 33 in accordance with the second exemplary embodiment of the present invention.

Sensor chip 33 of this exemplary embodiment is different from sensor chip 13 of the first exemplary embodiment in that an opening of through hole 38 does not have a circular shape but has a shape having a plurality of hole diameters, and in that the length direction of a longer hole diameter than the shortest length of the hole diameter of through hole 38 is parallel to solution flow direction H1.

As shown in Fig. 12, sensor chip 33 includes holding plate 32 having first surface 61 (upper surface) and second surface 62 (lower surface), and holding plate 32 is provided with a plurality of through holes 38 communicating between the first surface and the second surface. Furthermore, recess portion 29 is formed at the second surface side, that is, at a lower side of holding plate 32.

In this exemplary embodiment, holding plate 32 of sensor chip 33 has a double-layered structure including boron-doped layer 30 and silicon layer 31, and is covered with insulating film 43 such as a silicon dioxide film and a silicon nitride film.

Next, the shape of sensor chip 33 and arrangement of through holes 38 in this exemplary embodiment are described in detail.

Fig. 13 is a schematic plan view of holding plate 32 of sensor chip 33 in the sensor device in accordance with the second exemplary embodiment of the present invention.

As shown in Fig. 13, holding plate 32 of sensor chip 33 is formed in a square shape, and provided with nine through holes 38 thereon. Nine through holes 38 are arranged such that a shape drawn by line segments H2 connecting the centers of adjacent through holes 38 is a square shape. Each of nine through holes 38 has a shape having a plurality of hole diameters instead of having a circular shape at an opening on the first surface of holding plate 32.

Herein, the term "having a plurality of hole diameters" means that a distance from the center to the outer periphery of through hole 38 is not one distance. That is to say, the shape of the opening of through hole 38 is not a perfect circle but is a polygonal shape or a random shape.

In an example shown in Fig. 13, the shape of the opening of through hole 38 is a square of which the length of one side is "r". When the shape of the opening of through hole 38 is a square, longest length R1 of the opening of through hole 38 is a length of a diagonal line. A plurality of through holes 18 are arranged such that longest length R1 of the opening of through hole 38 corresponds to solution flow direction H1.

Note here that in an example shown in Fig. 13, a plurality of through holes 38 are formed in such a manner that longest length R1 of the opening of through hole 38 corresponds to solution flow direction H1. However, the present invention is not necessarily limited to this example. The direction of the hole diameter that is longer than the shortest length "r" of the hole diameters of through hole 38 may correspond to solution flow direction H1, and the possible length of the hole diameter is longer than "r" and not longer than √2r (= R1). That is to say, the direction of the shortest length "r" of the hole diameters of through hole 38 is not parallel to the direction of solution flow direction H1.

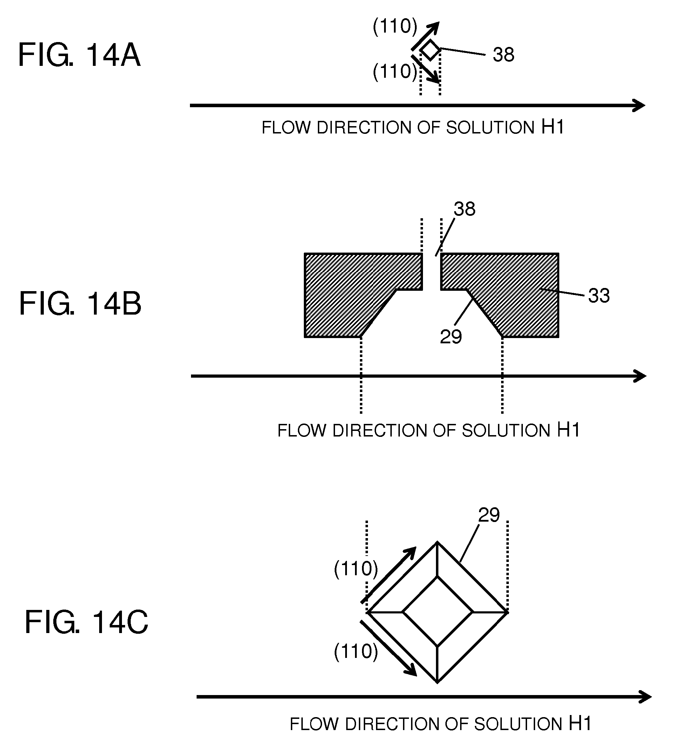

Fig. 14A is a plan view showing a configuration of through hole 38 of the sensor device in accordance with the second exemplary embodiment of the present invention, Fig. 14B is a sectional view showing a configuration of sensor chip 33, and Fig. 14C is a bottom view showing a configuration of recess portion 29.

As shown in Figs. 13, 14A and 14B, in this exemplary embodiment, holding plate 32 is formed in a square shape including a silicon (100) direction in one side. The shape of the opening of through hole 38 is formed in a square shape including a silicon (110) direction in one side. The direction of diagonal line R1 of the square shape of the opening of through hole 38 is parallel to solution flow direction H1.

Furthermore, as shown in Fig. 14C, the planar shape of recess portion 29 when holding plate 32 of sensor chip 33 is seen from the lower part is a square shape including the silicon (110) direction in one side, and the direction of one diagonal line of the square shape is parallel to solution flow direction H1.

Herein, as shown in Fig. 13, an angle made by the silicon (110) direction of holding plate 32 of sensor chip 33 and flow direction H1 of a solution flowing over the first surface of holding plate 32 is denoted by A.

As shown in Fig. 11A, the same crystalline direction is present every 90° on the silicon (100) substrate. Therefore, angle A can be a value in the range of 0° < A < 90°, and an angle capable of obtaining the maximum effect mentioned below is 45°. As the angle A approaches 45°, the larger effect can be obtained.

In order to form the shape of the opening of through hole 38 in a square shape, it is preferable to use the (100) direction of the silicon substrate for one side of the square shape of holding plate 32 of sensor chip 33. In this case, it is desirable from the viewpoint of productivity that a waste region in the substrate can be minimized.

That is to say, when the shape of sensor chip 33 is made to be a square shape that is the same shape as the opening of through hole 38, a plurality of sensor chips 33 can be formed from one substrate in the production of sensor chips 33 such that a waste region is a minimum. Thus, productivity can be improved. Furthermore, it is possible to effectively use a substrate used for production of sensor chip 33, which can contribute to the reduction of cost.

Furthermore, it is desirable in productivity that the (100) direction is used for one side of sensor chip 33 because the substrate can be formed such that a waste region is minimum.

However, when the direction other than the (110) direction is selected for one side of sensor chip 33, when a wafer is diced into individual sensor chip 33, the workability by blade dicing is lowered and it is difficult to form individual unit of sensor chip 33. However, when individual unit of sensor chip 33 is formed by dicing using a laser or etching between sensor chips 33, the workability of sensor chip 33 can be improved, and, for example, an arbitrary shape such as circle can be obtained.

Note here that difference in the processing methods by dicing can be determined by observing the side surface of sensor chip 33.

On the other hand, when sensor chip 33 has a square shape having the (110) direction in one side, it is preferable that line segments H2 connecting the centers of adjacent through holes 38 and one side of sensor chip 33 are not parallel to each other but they make a certain angle. However, when they are not parallel to each other, it is difficult to dispose through holes 38 on the entire surface of sensor chip 33, thus making it difficult to reduce the size of sensor chip 33. On the other hand, workability by blade dicing is improved. It is desirable that the shape of sensor chip 33 is selected by considering these effects comprehensively.

In an example shown in Fig. 13, sensor chip 13 is formed in a shape having one side in the (100) direction, and the shape of the opening of through hole 38 is a square. At this time, since through holes 38 are arranged such that one side of the square of the opening of each through hole 38 is rotated at 45° with respect to one side of sensor chip 13, it is possible to reduce the number of through holes 18 formed on the same silicon (110) direction. The advantageous effect thereof is described in detail.

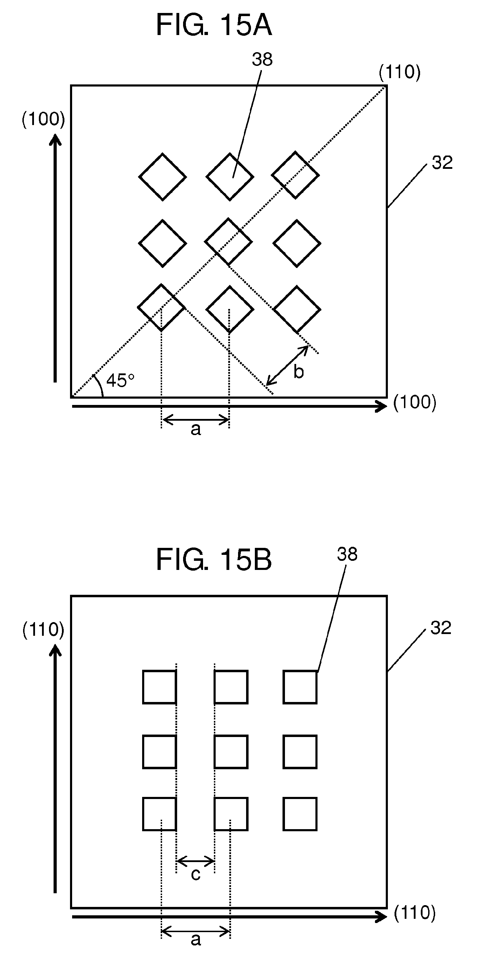

Fig. 15A is a schematic plan view for illustrating a configuration of sensor chip 33 in the sensor device in accordance with the second exemplary embodiment of the present invention, and Fig. 15B is a schematic plan view for illustrating another example of a configuration.

In an example of Fig. 15A, "a" denotes a distance of a line segment connecting the centers of adjacent through holes 38, and "b" denotes a length of a region in which through holes are not formed between adjacent through holes 38 in the silicon (110) direction when one side of holding plate 32 of sensor chip 33 is in the silicon (100) direction.

Furthermore, in an example of Fig. 15B, "a" denotes a distance of a line segment connecting the centers of adjacent through holes 38, and "c" denotes a length of a region in which through holes are not formed between adjacent through holes 38 in the silicon (110) direction when one side of holding plate 32 of sensor chip 33 is in the silicon (110).

At this time, the relation between length "b" and "c" satisfies: b > c. Even if distance "a" of the line segment connecting the centers of adjacent through holes 38 is the same, in the configuration of Fig. 15A, the number of through holes 38 formed in the silicon (110) direction can be reduced. As a result, since structurally weak through holes 38 are not continuously arranged, the structural strength is increased, and therefore cracking of sensor chip 33 can be reduced. Therefore, it is possible to accurately measure ionic current values of a cell, and the improvement of the success rate of measurement can be expected.

Furthermore, even when the interval "a" of the centers of through holes 38 is reduced, when sensor chip 13 has one side in the (100) direction (configuration shown in Fig. 15A), the strength of the sensor chip can be maintained as compared with the case where sensor chip 13 has one side in the (110) direction (configuration shown in Fig. 15B), and therefore higher density and smaller size of the sensor chip can be achieved. Thus, the number of sensor chips that can be produced from the same area is increased, which can contribute to the reduction of cost of the sensor chip.

Next, a method of producing sensor chip 33 used in the sensor device in this exemplary embodiment is described.

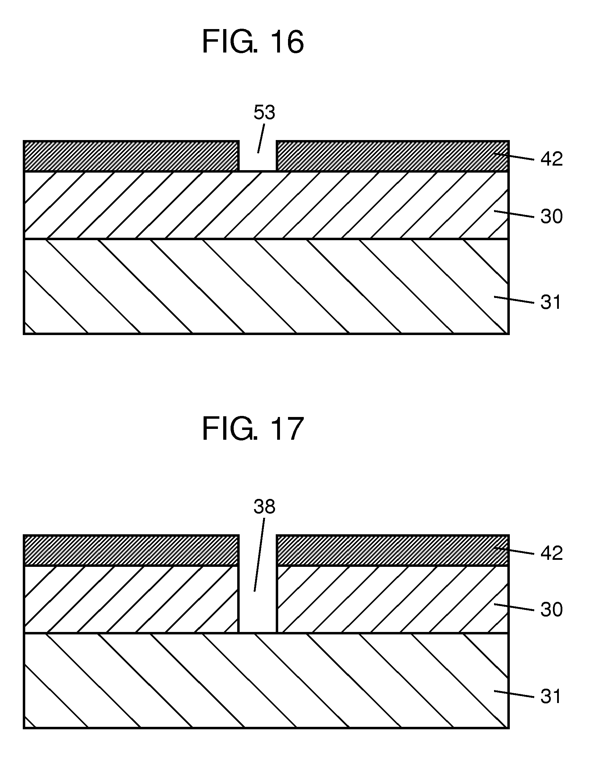

Figs. 16 to 19 are views for illustrating a manufacturing method of sensor chip 33 in the sensor device in accordance with the second exemplary embodiment of the present invention.

In this exemplary embodiment, square-shaped sensor chip 33 is produced by using the silicon (100) substrate including silicon layer 31 having boron-doped layer 30 in a part of the thickness direction. Herein, a square shape having one side of sensor chip 33 in the (100) direction (the configuration shown in Fig. 15A) is produced.

Boron-doped layer 30 functions as a stop layer when silicon is wet-etched with an alkaline solution. Thus, the highly accurate sensor chip 33 shape can be achieved. The thickness of boron-doped layer 30 is 1 to 30 µm, and the concentration of boron is not lower than 2 x 1019 (/cm³).

It is suitable that an element is injected into silicon layer 31 by an ion injection method. The ion injection method is a method of electrically accelerating ions and allowing ions to collide with an object, thereby injecting a specific element into the substrate. The controllability of the concentration distribution in the depth direction is excellent. Thus, it is possible to add a desirable element with higher accuracy. Other methods include an injection method using plasma, and thermal diffusion such as vapor phase diffusion or solid phase diffusion. When these injection methods of elements are carried out, concentration gradient occurs in the elements which are added to the inside of silicon. Furthermore, an element is also added by thermal diffusion to insulating film 43 such as a silicon dioxide film and a silicon nitride film formed by thermal oxidation, and therefore the concentration gradient occurs.

Firstly, as shown in Fig. 16, first resist mask 42 is formed on the first surface above boron-doped layer 30 by photolithography, and mask hole 53 having a size (0.5 µm to 5 µm) that is similar to that of through hole 38 is formed. The shape of mask hole 53 is a circular shape.

Next, as shown in Fig. 17, by using a Bosch process capable of vertical processing of silicon, through hole 38 is formed. Through hole 38 is formed so as to have a length that is not less than the thickness of boron-doped layer 30. In general, vertical through hole 38 having a depth of 1 to 50 µm is formed.

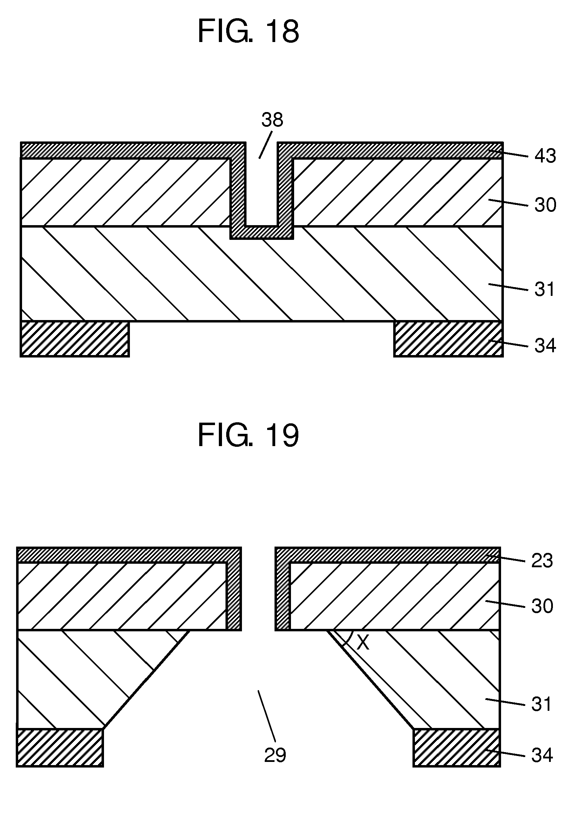

Next, as shown in Fig. 18, insulating film 43 made of a silicon dioxide film and a silicon nitride film is coated on the inside of through hole 38 by using a method such as LPCVD (Low Pressure Chemical Vapor Deposition).

As insulating film 43, any materials and forming methods may be employed as long as they are resistant to alkaline etching. Thereafter, by using photolithography, second resist mask 34 is formed on the second surface of silicon layer 31 opposite to the side on which boron-doped layer 30 is formed.

Then, as shown in Fig. 19, by using anisotropic wet etching of silicon with an alkaline solution such as KOH or TMAH solution, recess portion 29 is formed. The depth of recess portion 29 can be controlled by the etching time. In this exemplary embodiment, since boron-doped layer 30 is formed at the first surface side of sensor chip 33, etching stop can be regulated, and therefore the shape of sensor chip 33 can be controlled with high accuracy.

With this etching, from the difference in the etching rate by the orientation of silicon, the shape of recess portion 29 can be made into a pyramid shape surrounded by silicon (111) planes. At this time, angle X made by the side surface of silicon layer 31 and boron-doped layer 30 is about 54°.

In the case of silicon wet etching with an alkaline solution, the etching rate generally satisfies: silicon (100) > silicon (110) >> silicon (111). In other words, a pyramid shape of recess portion 29 has a square shape in which one side of the upper surface of the pyramid shape is one side in the (110) direction when it is vertically seen with respect to the substrate surface as shown in Fig. 14C. Wet etching of silicon with an alkaline solution facilitates one-time treatment of a plurality of layers and simplifies a device, and therefore, it has a merit in terms of cost.

Besides, recess portion 29 can be formed by using gas etching capable of etching silicon with SF₆, CF₄, Cl₂, XeF₂, or the like. However, in these cases, difference in the etching rate due to the orientations of silicon, recess portion 29 does not have a clear pyramid shape but has a relatively round shape.

Note here that, if necessary, as shown in Fig. 12, after an etch mask is selectively removed with respect to silicon layer 31, insulating film 43 such as a silicon dioxide film and a silicon nitride film is formed on entire sensor chip 33. When the thickness of insulating film 43 is not less than 300 nm, the above-mentioned effect can be achieved. However, since the shape of an opening of through hole 38 approaches a rectangular shape (square shape) as the thickness of the oxide film is larger, an effect with respect to the suction of specimen 19 or circulation of a drug solution is remarkably exhibited. Herein, the thickness of insulating film 43 is made to be 300 to 2000 nm. In this case, it is desirable that formation is carried out at temperatures not higher than the softening point of insulating film 43.

The growing rate of the silicon dioxide film depends on the orientation, and generally satisfies: silicon (110) > silicon (100). When the silicon dioxide film by thermal oxidation is formed as insulating film 43, as insulating film 43 is being formed, through holes 38 having a circular shape cannot easily maintain a circular shape and the shape of through hole 38 becomes a square shape. In this way, the shape of the opening of through hole 38 can be made into a square. That is to say, the shape of an opening of through hole 38 can be formed in a square shape having the silicon (110) direction in one side when it is vertically seen with respect to the substrate.

In this exemplary embodiment, in particular, through hole 38 formed in sensor chip 33 has a plurality of hole diameters on the opening, the length direction of the longer hole diameter than the shortest length of the hole diameter of a plurality of hole diameters is parallel to the solution flow direction. Therefore, the suction of specimen 19 is efficiently carried out, and the success rate of measurement can be improved.

In particular, when the shape of the opening of through hole 38 is formed in a square shape, the direction of diagonal line R1 that is the longest length in the hole diameters corresponds to flow direction H1 of a solution flowing over the first surface of sensor chip 33. Therefore, suction of specimen 19 is easily carried out efficiently, and the success rate of measurement can be improved.

Furthermore, the flow direction of the solution that flows below the second surface of sensor chip 33, that is, a direction in which a solution necessary for measurement of, for example, antibiotics is introduced, corresponds to the direction of the diagonal line of a recess shape of recess portion 29. Therefore, the circulation of the antibiotics becomes smooth and the success rate of measurement can be improved.

Furthermore, a square shape having the silicon (100) direction in one side is used as holding plate 32 of sensor chip 33, the shape of the opening of through hole 38 is a square shape, and the direction of diagonal line R1 of the square shape of the opening of through hole 38 is parallel to solution flow direction H1.

With the above-mentioned configuration, flow direction H1 of a solution that flows over the first surface of sensor chip 33, that is, an introducing direction of a solution including specimen 19 corresponds to the silicon (100) direction of sensor chip 33. Furthermore, at this time, the direction of diagonal line R1 of the square shape of the opening of through hole 38 also corresponds to the silicon (100) direction. That is to say, when measurement is carried out by introducing specimen 19, the direction of diagonal line R1 having the longest length of the square shape of through hole 38 corresponds to flow direction H1 of a solution that flows over the first surface of sensor chip 33, that is, the direction of specimen 19 is introduced. Consequently, suction of specimen 19 is easily carried out efficiently, and the success rate of measurement can be improved.

Similarly, the planar shape of recess portion 29 when holding plate 32 of sensor chip 33 is seen from the lower part is a square shape including the silicon (110) direction in one side, the direction of one diagonal line of this planar shape is parallel to solution flow direction H1.

With the above-mentioned configuration, the direction of the solution that flows below the second surface of holding plate 32 of sensor chip 33 corresponds to the silicon (100) direction of sensor chip 33. Furthermore, at this time, a diagonal line direction of the square shape of recess portion 29 corresponds to the silicon (100) direction.

That is to say, the flow direction of the solution that flows below the second surface of sensor chip 33, that is, a direction in which a solution necessary for measurement of, for example, antibiotics is introduced, corresponds to the direction of the diagonal line having the longest length of the square shape of recess portion 29. Therefore, the circulation of the antibiotics becomes smooth and the success rate of measurement can be improved.

Furthermore, by forming recess portion 29, the resistance of the flow passage can be suppressed. Thus, even when the thickness of sensor chip 33 is large, by reducing the resistance of the flow passage, the circulation efficiency of drug or suction of specimen 19 can be easily achieved.

Furthermore, as the additional effect, it is possible reduce cracking in sensor chip 33 and to improve the success rate of measurement. Since the centers of adjacent through holes 38 are not aligned in the silicon (110) direction and structurally weak through holes 38 are not continuously arranged on the cleavage plane of silicone, cracking in sensor chip 33 can be reduced. Thus, ionic current values of a cell as specimen 19 can be measured accurately, and improvement of the success rate of measurement can be expected.

Furthermore, even if the distance between the centers of through holes 38 is reduced, the strength of sensor chip 33 can be maintained, and higher density and smaller size of sensor chip 33 can be achieved. Thus, the number of sensor chips 33 produced from the same area is increased, which can contribute to the reduction of cost of sensor chip 33.

### THIRD EXEMPLARY EMBODIMENT

Hereinafter, a sensor device in accordance with a third exemplary embodiment of the present invention is described.

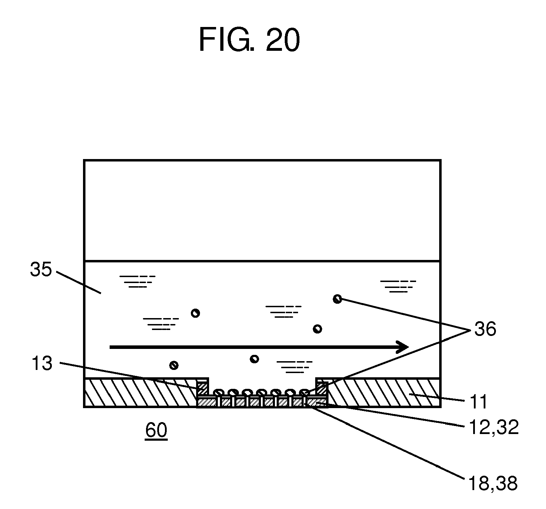

Fig. 20 is a schematic sectional view showing a configuration of a sensor device in accordance with the third exemplary embodiment of the present invention.

The same reference numerals are given to the same components described in the first and second exemplary embodiments and the description thereof is omitted. The first and second exemplary embodiments describe cell electrophysiological sensor 50 as an example of the sensor device, but the sensor deice of the present invention can be also used as substance identification sensor 60. A sensor device in the substance identification sensor is a device capable of adsorbing and holding specimen 36 including a bead including a probe, and the like, and measuring a sample such as a chemical substance, a biological substance, and an environment substance.

As shown in Fig. 20, substance identification sensor 60 in this exemplary embodiment includes mounting substrate 11, sensor chip 13, 33 which is mounted on mounting substrate 11 and has holding plate 12. Furthermore, flow passage 35, in which a solution can be allowed to flow along mounting substrate 11, is formed above sensor chip 13, 33. Then, sensor chip 13, 33 has a plurality of through holes 18, 38 formed from the first surface to the second surface.

Then, a solution including specimen 36 is allowed to flow in flow passage 35, and specimen 36 is allowed to be held in plurality of through holes 18, 38. At this time, by pressurizing from the first surface of sensor chip 13, 33 via through holes 18, 38, or depressurizing from the second surface of sensor chip 13, 33, specimen 36 and the solution are attracted to plurality of through holes 18, 38. Then, specimen 36 is sucked and held so as to block through holes 18, 38.

Then, in this state, fluorescence intensity, surface compositions, a surface state, and the like, can be measured.

### INDUSTRIAL APPLICABILITY

As mentioned above, a sensor device of the present invention has a special advantageous effect capable of adsorbing and holding a specimen in a through hole with high accuracy, and therefore it is useful in the fields of medicine, biotechnology, and the like, in which a high success rate of measurement is required.

### REFERENCE MARKS IN DRAWINGS

- 11: mounting substrate
- 12, 32: holding plate
- 13, 33: sensor chip
- 14: first electrode bath
- 14a: wall surface
- 14b: flow passage width
- 15: first electrode
- 16: second electrode bath
- 17: second electrode
- 18, 18a, 18b, 18c, 38: through hole
- 19, 36: specimen
- 20, 22, 31: silicon layer
- 21: silicon dioxide layer
- 23, 43: insulating film
- 24, 42: first resist mask
- 25, 53: mask hole
- 26: concave portion
- 27, 34: second resist mask
- 28: cavity
- 29: recess portion
- 30: boron-doped layer
- 35: flow passage
- 50: cell electrophysiological sensor
- 51, 61: first surface
- 52, 62: second surface
- 60: substance identification sensor

## Claims

1. A sensor device comprising:
a mounting substrate; and
a sensor chip held on the mounting substrate,
wherein the sensor chip includes a holding plate, and a plurality of through holes each penetrating through a first surface and a second surface opposite to the first surface of the holding plate,
a solution including a specimen is allowed to flow over the first surface of the holding plate, and the specimen is held in the plurality of through holes, and
directions of line segments connecting centers of adjacent through holes among the plurality of through holes are not parallel to a flow direction of the solution.

2. The sensor device of claim 1, wherein a shape of the holding plate is a square.

3. The sensor device of claim 1, wherein a (100) plane of a silicon substrate is used as the holding plate.

4. The sensor device of claim 3, wherein the flow direction of the solution flowing over the first surface of the holding plate corresponds to a (110) direction of the silicon substrate.

5. The sensor device of claim 1, wherein a line shape drawn by the line segments connecting the centers of the adjacent through holes is a regular polygon shape.

6. The sensor device of claim 5, wherein the regular polygon shape is a square.

7. The sensor device of claim 5, wherein the regular polygon shape is a regular triangle.

8. The sensor device of claim 1, wherein a length of the line segment connecting the centers of the adjacent through holes is not less than 20 µm.

9. A sensor device comprising:
a mounting substrate; and
a sensor chip held on the mounting substrate,
wherein the sensor chip includes a holding plate, and a plurality of through holes each penetrating through a first surface and a second surface opposite to the first surface of the holding plate,
a solution including a specimen is allowed to flow over the first surface of the holding plate, and the specimen is held in the plurality of through holes,
each of the plurality of through holes has a plurality of hole diameters, and
a length direction of a shortest hole diameter among the plurality of hole diameters is not parallel to a flow direction of the solution.

10. The sensor device of claim 9, wherein a length direction of a longest hole diameter among the plurality of hole diameters is parallel to the flow direction of the solution.

11. The sensor device of claim 9, wherein a shape of the holding plate is a square.

12. The sensor device of claim 9, wherein a (100) plane of a silicon substrate is used as the holding plate.

13. The sensor device of claim 12, wherein the length direction of the shortest hole diameter among the plurality of hole diameters is not parallel to a (100) direction of the silicon substrate.

14. The sensor device of claim 9, wherein a shape of the holding plate is a square, a (100) plane of a silicon substrate is used as the holding plate, and a diagonal line direction of the holding plate is parallel to a silicon (110) direction.

15. The sensor device of claim 9, wherein a shape of the holding plate is a square, a (100) plane of a silicon substrate is used as the holding plate, and a direction of one side of the holding plate is parallel to a silicon (110) direction.

16. The sensor device of claim 9 or 15, wherein line segments connecting centers of adjacent through holes are parallel to the flow direction of the solution.

17. The sensor device of claim 9, wherein a shape of an opening on the first surface of the through hole is a square.

18. The sensor device of claim 17, wherein a direction of a diagonal line of a square of the opening on the first surface of the through hole is parallel to a silicon (100) direction.

19. The sensor device of claim 17, wherein one side of the square of the opening on the first surface of the through hole is parallel to a silicon (110) direction.

20. The sensor device of claim 9, wherein the flow direction of the solution is parallel to the silicon (100) direction.

21. A sensor device comprising:
a mounting substrate; and
a sensor chip held on the mounting substrate,
wherein the sensor chip includes a holding plate, and a plurality of through holes each penetrating through a first surface and a second surface opposite to the first surface of the holding plate,
a first solution including a specimen is allowed to flow over the first surface of the holding plate, a second solution is allowed to flow over the second surface of the holding plate, and the specimen is held in the plurality of through holes,
the second surface of the holding plate is provided with a recess portion communicating to the plurality of through holes,
the recess portion has a plurality of hole diameters in a planar shape on the second surface of the holding plate, and
a length direction of a shortest hole diameter among the plurality of hole diameters of the recess portion is not parallel to a flow direction of the second solution.

22. The sensor device of claim 21, wherein a length direction of a longest hole diameter among the plurality of hole diameters is parallel to a flow direction of the second solution.

23. The sensor device of claim 21, wherein a shape of the holding plate is a square.

24. The sensor device of claim 21, wherein a (100) plane of a silicon substrate is used as the holding plate.

25. The sensor device of claim 24, wherein the length direction of the shortest hole diameter among the plurality of hole diameters is not parallel to a (100) direction of the silicon substrate.

26. The sensor device of claim 21, wherein a shape of the holding plate is a square, a (100) plane of a silicon substrate is used as the holding plate, and a diagonal line direction of the holding plate is parallel to a silicon (110) direction.

27. The sensor device of claim 21, wherein a shape of the holding plate is a square, a (100) plane of a silicon substrate is used as the holding plate, and a direction of one side of the holding plate is parallel to a silicon (110) direction.

28. The sensor device of claim 21, wherein a shape of an opening on the second surface of the recess portion is a square.

29. The sensor device of claim 28, wherein a direction of a diagonal line of the square of the opening on the second surface of the recess portion is parallel to a silicon (100) direction.

30. The sensor device of claim 28, wherein a direction of one side of the square of the opening on the second surface of the recess portion is parallel to the silicon (110) direction.

31. The sensor device of claim 21, wherein the flow direction of the second solution is parallel to the silicon (100) direction.
